# EUROPEAN PATENT APPLICATION

(11) **EP 1 557 174 A1**
(43) Date of publication of application: **27.07.2005**
(21) Application number: 03755689.1
(22) Date of filing: 02.10.2003
(51) Int. Cl.: A61K 38/17, A61K 45/00, A61P 9/00, A61P 19/08, A61P 25/00, A61P 43/00, C12Q 1/02, G01N 33/50, G01N 33/15

(54) **COMPOSITION CONTAINING EOSINOPHIL CATIONIC PROTEIN**

(30) Priority: 07.10.2002 JP 2002294071
(71) Applicant: JAPAN SCIENCE AND TECHNOLOGY CORPORATION, Kawaguchi-shi, Saitama 332-0012 (JP)
(72) Inventor: SENO, Masaharu, Okayama-shi, Okayama 703-8273 (JP); KURODA, Shun'ichi, Suita-shi, Osaka 565-0872 (JP); TANIZAWA, Katsuyuki 30-2, Kibougaoka 2-chome, Toyono-gun, Osaka 563-0214 (JP); KITAZOE, Midori Soreiyu Harumi 105, Okayama-shi, Okayama 700-0953 (JP); IWATA, Miki, Yawata-shi, Kyoto 614-8373 (JP); FUJITA, Toshitsugu, Ueno-shi, Mie 518-0842 (JP); BOKUI, Nobuyuki 6-7, Minamitsutsujigaoka-ohbadai, Kameoka-shi, Kyoto 621-0846 (JP)
(74) Representative: Calamita, Roberto
(86) International application number: PCT/JP2003/012680
(87) International publication number: WO 2004/030689

(57) **Abstract**

A therapeutic composition for a disease caused by a failure in the survival, proliferation and/or differentiation of a cell which contains eosinophil cationic protein and other components; and a medium composition for promoting the survival, proliferation and/or differentiation of a cell.

## Description

### Technical Field

The invention of this application relates to a composition containing eosinophil cationic protein. More particularly, this application relates to a therapeutic composition and a cell culture medium composition utilizing the novel physiological activity of eosinophil cationic protein, and a screening method by using the physiological activity of eosinophil cationic protein as an indicator.

### Background Art

Eosinophil cationic protein (hereinafter referred to as "ECP") is a protein whose expression is increased in accordance with the activation of an eosinophil, and is present in a basic granule (Proc. NatI. Acad. Sci. USA. 1986, 83 (10): 3146-3150). It is known that ECP has activities of killing parasites, neurotoxin, inhibition of lymphocyte proliferation, sterilization, histamine release, ribonuclease, shortening the clotting time, and the like (Nippon Rinsho 1993, 51 (3): 60 (606)). In particular, since its histamine releasing activity induces an allergic reaction, an antiallergic drug whose pharmacological action is to inhibit the ECP expression has been proposed (JP-T-2002-500506 (the term "JP-T" as used herein means a published Japanese translation of a PCT patent application)). In addition, it has been proposed that an eosinophil cell line including ECP is utilized for screening of a drug for asthma or an antiallergic drug (JP-A-05-111382).

As described above, it has been recognized that the physiological activity of ECP is toxicity or proliferation inhibition against bacteria or animal cells so far.

On the contrary, the inventors of this application found that ECP has a novel physiological activity such as the survival or differentiation of an animal cell.

An object of the invention of this application is to provide a novel composition utilizing the novel activity of ESP that the inventors found.

In addition, another object of this application is to provide a novel method for developing a remedy whose pharmacological action is to promote the survival or differentiation of a cell by using the novel activity of ECP as an indicator.

### Disclosure of the Invention

This application provides, as a first invention for achieving the foregoing objects, a composition which is a therapeutic composition for a disease caused by a failure in the survival, proliferation and/or differentiation of a cell characterized by containing eosinophil cationic protein and a pharmacological component.

In the composition of this first invention, a preferred embodiment is that the disease caused by a failure in the survival, proliferation and/or differentiation of a cell is a heart disease, bone disease or neurodegenerative disease.

Further, this application provides, as a second invention, a composition which is a medium composition for promoting the survival, proliferation and/or differentiation of a cell and containing eosinophil cationic protein and a cell biological component.

Further, this application provides, as a third invention, a screening method, which is a method of screening an active ingredient substance of a therapeutic composition for a disease caused by a failure in the survival, proliferation and/or differentiation of a cell, characterized by bringing a candidate substance into contact with a cell and specifying, as a target substance, a substance for promoting the survival and/or differentiation of a cell at the same level or higher than eosinophil cationic protein.

In this third invention, a preferred embodiment is that the cell is a nerve cell, bone cell, myocardial cell or fibroblast.

In other words, the inventors of this application found the following novel activities of ECP, which was conventionally considered to have physiological activities of cytotoxicity and inhibition of cell proliferation.
(1) Promotion of fibroblast proliferation and promotion of stress fiber formation
(2) Maturing of muscle fiber of a myocardial cell and increase in the number of heartbeats
(3) Improvement of survival rate of nerve cells in a low-serum medium or serum-free medium
(4) Promotion of fibroblast differentiation

In addition, the foregoing activities are inhibited or lost by an inhibitor against a substrate of low molecular weight G-protein Rho-kinase (ROCK) which plays an important role in the signal transduction pathway in a cell. Therefore, it was found that ECP affects the signal transduction pathway in a cell and provides the foregoing (1) to (4) activities.

### Brief Description of Drawings

Fig. 1 shows the results obtained by studying the effects of ECP on the proliferation of cell lines derived from normal tissues. The closed circles indicate BALB/c 3T3 cells, the open triangles indicate A10 cells, the open squares indicate HC-11 cells, and the open circles indicate HUVEC cells.
Fig. 2 shows the results obtained by studying the dose-dependent effects of ECP on the proliferation of BALB/c 3T3 cells.
Fig. 3 shows the results obtained by studying the time-course effects of ECP on the proliferation of BALB/c 3T3 cells. The closed circles indicate EPC, the closed squares indicate RNase and the open squares indicate the control.
Fig. 4 shows the results (phase-contrast microscopic images) obtained by studying the effects of ECP on the proliferation of BALB/c 3T3 cells in a low-serum medium.
Fig. 5 shows the results (confocal laser scanning microscopic images) obtained by studying the effects of ECP on the formation of BALB/c 3T3 cytoskeletal molecule in a low-serum medium.
Fig. 6 shows the results (confocal laser scanning microscopic images) obtained by studying the effects of ECP + bFGF on the formation of BALB/c 3T3 cytoskeletal molecule in a low-serum medium.
Fig. 7 shows the results (confocal laser scanning microscopic images) obtained by studying the effects of ECP and a ROCK inhibitor on the cytoskeleton formation of BALB/c 3T3 cells.
Fig. 8 shows the results (confocal laser scanning microscopic images) obtained by studying the effects of ECP on the cytoskeleton formation in myocardial cell derived from a newborn rat.
Fig. 9 shows the results obtained by studying the effects of ECP on the number of heartbeats of myocardial cell derived from a newborn rat.
Fig. 10 shows the results obtained by studying the effects of ECP and a ROCK inhibitor on the number of heartbeats of myocardial cell.
Fig. 11 shows the results obtained by studying the effects of ECP on the survival of nerve like PC12 cells in a serum-free medium. The columns 1, 2, 3, and 4 correspond to the mean values in the cases of before ECP addition, addition of ECP at 0 ng/ml, 10 ng/ml and 1000 ng/ml, respectively.
Fig. 12 shows the results obtained by studying the effects of ECP on the alkali phosphatase activity in MC3T3-E1 cells derived from calvarial bone of a rat.

### Best Mode for Carrying Out the Invention

The invention of this application is based on the following new findings. Hereunder, with regard to each invention of this application, embodiments will be explained in detail.

ECP used in each invention of this application can be isolated from a cell (leukocyte cell or hematopoietic stem cell) of a variety of mammals such as human by a known method. In addition, it can be also produced by chemical synthesis using a known solid-phase peptide synthetic method based on the amino acid sequence (human ECP: GenBank/X15161, chimpanzee: GenBank/AF294028, gorilla: GenBank/U24097) or the like. Further, it can be obtained as a recombinant ECP by expressing a polynucleotide encoding each peptide in an in vitro transcription/translation system or an appropriate host-vector system. The polynucleotide (e.g., ECP cDNA) can be obtained by a method of screening an existing cDNA library with the use of an oligonucleotide probe produced based on the nucleotide sequence information in the foregoing GenBank database, or a known method such as the RT-PCR method using an oligonucleotide primer.

For example, in the case of producing a recombinant ECP in an in vitro transcription/translation system, the foregoing polynucleotide is inserted into a vector with an RNA polimerase promoter to produce an expression vector, and this vector is added to an in vitro translation system such as a rabbit reticulocyte lysate or a wheat germ extract containing an RNA polymerase corresponding to the promoter. As the RNA polymerase promoter, T7, T3, SP6 and the like can be exemplified. As the vector containing such an RNA polymerase promoter, pKA1, pCDM8, pT3/T7 18, pT7/3 19, pBluescript II and the like can be exemplified.

In the case of expressing such a recombinant ECP in a microorganism such as E. coli, a recombinant expression vector is produced by recombining the foregoing DNA fragment into an expression vector having an origin capable of replication in a microorganism, a promoter, ribosome binding site, DNA cloning site, terminator or the like, and a fusion peptide is isolated from the culture. As the expression vector for E. coli, a pUC system, pBluescript II, pET expression system, pGEX expression system and the like can be exemplified.

In the case of expressing such a recombinant ECP in a eukaryotic cell, a recombinant expression vector is produced by inserting the foregoing fusion polynucleotide into an expression vector for a eukaryotic cell having a promoter, splicing site, poly(A) addition site or the like, and the recombinant vector is introduced into a eukaryotic cell, whereby a fusion peptide can be expressed in a transformed eukaryotic cell. As the expression vector, pKA1, pCDM8, pSVK3, pMSG, pSVL, pBK-CMV, pBK-RSV, EBV vector, pRS, pcDNA3, pMSG, pYES2 and the like can be exemplified. As the eukaryotic cell, a cultured mammalian cell such as a monkey kidney cell COS7 and Chinese hamster ovary cell CHO, budding yeast, dividing yeast, silkworm cell, African clawed frog egg cell and the like are usually used, however, any eukaryotic cell may be used so long as it is able to express a desired protein.

For the introduction of the expression vector into a host cell, a known method such as an electroporation method, a calcium phosphate method, a liposome method, and a DEAE dextran method can be employed.

In order to isolate or purify a recombinant ECP from the culture after expressing the fusion peptide in a prokaryotic cell or a eukaryotic cell, it can be performed by combining known separation procedures. For example, these procedures include treatment with a denaturation reagent such as urea or with a surface active agent, ultrasonic treatment, enzymatic digestion, salting-out and solvent precipitation method, dialysis, centrifugation, ultrafiltration, gel filtration, SDS-PAGE, isoelectric focusing electrophoresis, ion exchange chromatography, hydrophobic chromatography, affinity chromatography, reversed phase chromatography and the like.

Incidentally, ECP is a secretory protein, and in the case of, for example, human ECP, 23-amino acid sequence at the N-terminal side is a secretory signal sequence. Therefore, in the case where a recombinant ECP is expressed in a prokaryotic cell or a eukaryotic cell, it is preferred that an active region at the C-terminal side from the secretory signal sequence (e.g., C-terminal residues from Arg-28) is expressed in consideration of the recovery efficiency of the recombinant ECP from the culture.

The composition of the first invention is a medical composition characterized by containing the foregoing ECP and a pharmacological component, and is used for a cure of a human disease caused by a failure in the survival, proliferation and/or differentiation of a cell. In other words, such a disease is developed by an aberration in a normal lifecycle (proliferation, differentiation, apoptosis or the like) of a variety of tissue cells of the body, and a variety of diseases caused by heredity or various cytotoxic substances can be used as a target. In particular, it can be used for a complete cure, remission or improvement of symptom of a heart disease, bone disease or neurodegenerative disease. Examples of the heart disease include cardiac infarction caused by such as aberration or degeneration of myocardial cytoskeleton formation, myocarditis, cardiomyopathy (such as dilated cardiomyopathy, hypertrophic cardiomyopathy, restrictive cardiomyopathy, and obliterative cardiomyopathy), myocardial fibrosis and the like. Examples of the bone disease include particularly osteoporosis caused by a failure in differentiation of a bone-forming cell (osteoblast) and periodontal disease. In addition, this therapeutic composition can be used for osseous tissue regeneration after fracture. Examples of the neurodegenerative disease include Alzheimer disease caused by degeneration or apoptosis of a nerve cell, dementia senilis, Down syndrome, Parkinson's disease, Creutzfeldt- Jakob disease, amyotrophic lateral sclerosis, neuromyopathy and the like.

The "pharmacological components", which is a component of the medical composition of the first invention means firstly, a variety of carriers to be used in the usual drug production. The carrier can be appropriately selected from a wide range according to the types of a target disease or a dosage form of a drug, however, it is desired that the medical composition of this invention is in a unit dosage form which can be administered orally or by injection. Particularly, in the case of administration by injection, topical injection, intraperitoneal injection, selective intravenous infusion, intravenous injection, hypodermic injection, organ perfusate infusion or the like can be adopted.

An oral liquid preparation such as a suspension or a syrup can be produced by using water, a saccharide such as sucrose, sorbitol and fructose, a glycol such as polyethylene glycol, an oil such as sesame oil and soybean oil, an antiseptic such as alkyl p-hydroxybenzoate, a flavor such as strawberry flavor, peppermint, etc.

A powder, pill, capsule, and tablet can be formulated by using a diluent such as lactose, glucose, sucrose and mannitol, a disintegrator such as starch and sodium alginate, a lubricant such as magnesium stearate and talk, a binder such as polyvinyl alcohol, hydroxypropyl cellulose, and gelatin, a surface active agent such as a fatty acid ester, a plasticizer such as glycerin, etc. A tablet and a capsule are a preferred unit dosage form for the composition of this invention in that it is easy to administer. In the production of a tablet or a capsule, a solid pharmaceutical carrier is used.

In addition, a solution for injection can be formulated by using a carrier comprising a salt solution, a glucose solution or a mixture of a salt water and a glucose solution, a variety of buffers or the like. Alternatively, it is formulated in a powder form, and an injection solution may be prepared by mixing the powder with the foregoing liquid carrier at the point of use.

The administration amount of the medical composition of this invention varies depending on the age or body weight of a patient, symptom, administration route or the like, however, the amount that allows the blood concentration of ECP to be about 10 nmol to 0.1 mmol, preferably about 5 nmol to 0.5 mmol may be administered.

A second pharmacological components is a component for allowing ECP to be in a form that can be introduced into a cell. For example, this polypeptide is mixed with a solution which does not change the structure or the function of this polypeptide and is pharmacologically acceptable, whereby the composition can be prepared. Such a composition can be introduced into a target cell by, for example, an intracellular introduction method by a microinjection method or by an intracellular introduction method using a lipid (e.g., BioPORTER (Gene Therapy Systems, USA), or Chariot (Active Motif, USA), or the like).

A third pharmacological components is a component for allowing a polynucleotide encoding ECP to be in a form that can be introduced into a cell. In other words, it may be performed by incorporating the polynucleotide into an expression vector for a eukaryotic cell, and incorporating this vector into, for example, a hollow nanoparticle presenting a biorecognizable molecule, retrovirus, adenovirus, adeno-associated virus or the like. Such a composition can be introduced into a target cell in the body by a method of gene therapy.

The second invention of this application is a medium composition containing eosinophil cationic protein and a cell biological component, and can be used for promoting the survival, proliferation and/or differentiation of a cultured cell.

The "cell biological component" is a component essential for the survival, proliferation, differentiation or the like of a cell and specifically, it is a component constituting a usual medium for an animal cell. Specific examples include a buffer (phosphate, sodium bicarbonate, carbon dioxide gas or the like), a salt, glucose, a vitamin, an organic substance such as an amino acid, serum (fetal bovine serum: FBS), a nutritional factor (growth factor in serum: GFS) and the like. The medium composition of this invention can be produced by appropriately mixing such a component and ECP. The content of each component can be set at the same level as the usual culture medium for an animal. The content of ECP can be arbitrarily set depending on the type of a cell or the object, however, it can be set to about 0.001 to 10 µ per 1 ml of medium.

As the "cell", a cell which is usually used as a target for an animal cell can be used, however, particularly a nerve cell, bone cell, myocardial cell, fibroblast and the like are preferred. In addition, such a cell may be a primary culture cell isolated from an animal tissue, passage culture cell, established cell line, or transformed cell into which a foreign gene has been introduced. "Culture" can be carried out in a floating culture system in the case of a floating cell, and in a monolayer culture system or three-dimensional culture system in the case of an adhesive cell. In addition, it may be carried out in a hollow fiber culture system in a hollow polymeric tube.

The culture using the medium composition of the second invention allows a cultured cell to survive over a long period of time, or to be differentiated for expressing a function. Therefore, the culture system using this composition is useful in elucidating a cell biological event such as the survival, cytoskeleton formation, proliferation or differentiation of a cell, or the signal transduction cascade regulating these events (especially, Rho kinase pathway). In addition, since this composition allows a cell to be differentiated in a state of expressing a function, or to survive over a long period of time, this composition can be utilized in the production (bioreactor or the like) of a useful substance.

Further, the medium composition of this invention allows a nerve cell or the like to survive over a long period of time even in a low-serum or a serum-free condition by containing ECP. Therefore, because a cell function can be analyzed in a condition of low protein or free of protein except for ECP, it is useful in a system for screening a protein factor, etc. which affects a specific function of a cell.

The third invention of this application is a screening method for specifying a novel factor other than ECP, which has an action of promoting the survival, proliferation and/or differentiation of a cell. In other words, a candidate substance is brought into contact with a cell, and the survival time or differentiation state of the cell is measured. Then, in the case where the measurement value is equal to or higher than the measurement value when ECP was brought into contact with a cell, the tested candidate substance can be determined to be a target factor. The survival of a cell can be measured by a known method such as the trypan blue staining method. The proliferation of a cell can be measured by counting the number of living cells after being cultured for a predetermined period. The differentiation of a cell can be measured by the immunostaining method using a marker specific to a differentiated cell as a target, a Western blot analysis, the RT-PCR method or the like.

The cell is a cell for prolonging its survival time or a cell for promoting its proliferation or differentiation by the contact with ECP, and specifically, a nerve cell, bone cell, myocardial cell, fibroblast and the like are preferred. In addition, such a cell may be a primary culture cell isolated from an animal tissue, passage culture cell, established cell line, or transformed cell into which a foreign gene has been introduced. Such a cell can be examined under the same condition as in a usual animal cell culture. In the case of examining the survival of a cell, it is preferred that the cell is cultured under a condition without serum or a growth factor. Further, such a cell may be a cell present in an individual animal.

The "candidate substance" is, for example, an unknown or known organic or inorganic compound, protein, peptide, polynucleotide, oligonucleotide or the like. In the case where such a candidate substance is brought into contact with a cell, a method of adding the candidate substance to a medium of cultured cells, a method of introducing the candidate substance into a cultured cell or a cell present in an individual animal (microinjection or an intracellular introduction method using a lipid) or the like can be adopted. In addition, in the case where the candidate substance is a polynucleotide or an oligonucleotide, the expression vector thereof may be transfected into a cell by a known method or an individual animal may be infected with a virus vector in accordance with a method of gene therapy.

Novel factors specified by this screening method can become an effective component of a therapeutic composition or a medium composition solely or in combination with ECP.

Hereunder, by showing the results of experiment and research performed in order to confirm the novel physiological activity of ECP as Examples, the invention of this application will be explained in more detail and specifically, however, the invention of this application is by no means limited to the following examples.

### Examples

### Example 1: Effects of ECP on proliferation of cell line derived from normal tissue

The effects of ECP on the proliferation of a mouse fibroblast cell line (BALB/c 3T3), aortic smooth muscle cell line (A10), mouse mammary epithelial cell line (HC-11) and human umbilical vessel endothelial cell line (HUVEC) were analyzed in the presence of 10% FBS. First, in DMEM culture medium containing 10% FBS in a 96-well culture plate, each cell line was inoculated at a concentration of 500 cells/well. Then, the cells were cultured for 24 hours in an incubator in which CO₂ was maintained at 5%. Then, the respective concentrations of ECP and RNaseA (0 to 10 µM) were added, and the culture was continued for 48 hours in the same incubator. Then, 3-(4,5-dimethylthiazol-2-yl)-diphenyl-tetrazolium bromide (MTT) was added, and the proliferation rate of each cell line was obtained.

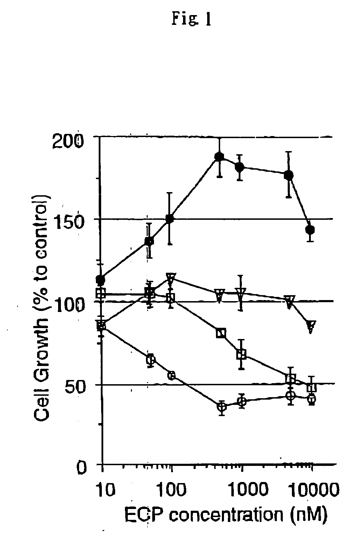

The results were shown in Fig. 1. As is clear from the Fig. 1, it was confirmed that, with regard to BALB/c 3T3 cell line, the cell proliferation was promoted by adding ECP. On the other hand, with regard to the other cell lines, the proliferation was not promoted or the proliferation was inhibited by ECP.

### Example 2: Effects of ECP on proliferation of BALB/c 3T3 cell

In DMEM culture medium containing 10% FBS in a 96-well culture plate, BALB/e 3T3 A31-K cells were inoculated at a concentration of 1000 cells/well, and after 24 hours, ECP or RNaseA was added to the culture solution to a final concentration of 1 µM, respectively. After the culture was continued for 48 hours, the number of living cells was counted.

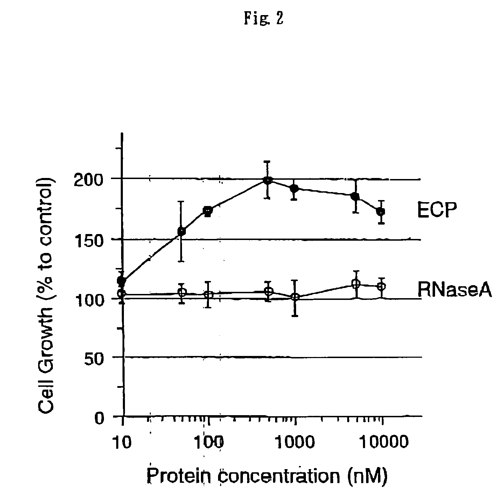

The results are as shown in Fig. 2. ECP significantly promoted the cell proliferation at any concentration. On the other hand, effects of RNaseA on promoting cell proliferation could not be observed.

### Example 3: Time-course effects of ECP on proliferation of BALB/c 3T3 cell

In DMEM culture medium containing 10% FBS in a 96-well culture plate, BALB/c 3T3 cells were inoculated at a concentration of 1000 cells/well. After the cells were cultured for 24 hours, 1 µM ECP or 1µM RNaseA was added to the culture solution to a final concentration of 1 ng/ml, respectively. The culture was continued for 4 days, and the number of living cells was counted for every 24 hours. Incidentally, the culture medium was changed on day 2 of the culture.

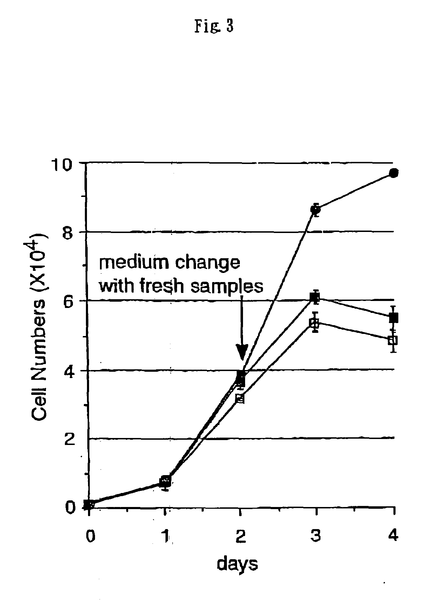

The results are as shown in Fig. 3. With regard to the condition of ECP addition, after the medium was changed on day 2 of the culture, the proliferation of cells was significantly promoted.

### Example 4: Effects of ECP on proliferation of BALB/c 3T3 cell under condition of low-serum medium

In DMEM culture medium containing 10% FBS in a 24-well culture plate, BALB/c 3T3 cells were inoculated at a concentration of 2 x 10⁴ cells/well, and cultured. After 24 hours, when the cultured cells became stable, the medium was changed to DMEM culture medium containing 0.5% FBS, then the culture was continued. After 24 hours, 1 µM ECP or RNaseA, and 60 pM bFGF were added to the culture solution in combination, respectively, then the culture was continued for an additional 48 hours. Then, the cells cultured under the respective conditions were observed with a phase-contrast microscope (x 20 objective lens).

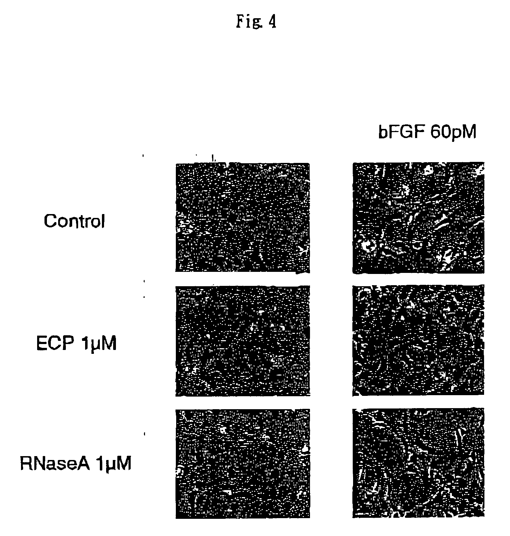

The results are as shown in Fig. 4. The cells were proliferated with bFGF solely, however, a more significant effect on cell proliferation was observed under the condition of bFGF + ECP.

### Example 5: Effects of ECP on BALB/c 3T3 cytoskeletal molecule under condition of low-serum medium

A round cover glass with a diameter of 15 mm which had been sterilized in advance was placed in a 24-well culture plate, 1x phosphate buffer (PBS) containing 0.1% gelatin was added to each well so that the cover glass was sufficiently dipped in the solution. Then, the plate was left at room temperature for 30 minutes, whereby the cover glass was coated with gelatin. Then, each well was filled with DMEM culture medium containing 10% FBS, and BALB/c 3T3 cells were inoculated at a concentration of 2 x 10⁴ cells/well and cultured. After 24 hours, the medium was changed to DMEM culture medium containing 0.5% FBS. After an additional 24 hours, 1 µM ECP or RNaseA was added to the culture solution, respectively. After 6 hours, the cover glass on which BALB/c 3T3 cells were grown, was taken out from the well, washed 2 to 3 times with 1x PBS, then dipped in 4% formaldehyde (Wako Pure Chemical Industries, Ltd.) at room temperature for 10 minutes, whereby the cells were fixed. Then, the cover glass was washed 2 to 3 times with 1x PBS, dipped in 1x PBS containing 0.1% Trton-X100 for 10 minutes, whereby the cell membranes were lysed. Then, the cover glass was washed 2 to 3 times with 1x PBS, dipped in 0.5% BSA/PBS and left at room temperature for 30 minutes, whereby a nonspecific adsorption region was blocked with BSA in this operation. A primary antibody was diluted to 11 µg/ml with PBS, and the cells were dipped in this antibody dilution for 30 minutes to perform a primary antibody reaction. As the primary antibody, three types: F-actin, vinculin and a mixture of F-actin and vinculin were used.

Then, every 5 minutes, the medium was changed and washing was performed with fresh 1x PBS. As a secondary antibody, RITC-conjugated goat anti-mouse IgG antibody (CHEMICON) and 10 µM FITC-phalloidin/methanol (Molecular Probes) were mixed in 1x PBS so as to yield a final concentration of 10 µg/ml and 100 nM, respectively, and the cells were dipped in this antibody dilution for 30 minutes to react with each other. Then, every 5 minutes, the medium was changed and washing was performed with fresh 1x PBS. Then, a solution of an equivalent amount of 1x PBS and glycerol was dropped on a slide glass and the foregoing round cover glass was placed thereon, and observation was performed with a confocal laser scanning microscope (MRC-1024, Bio-Rad Co).

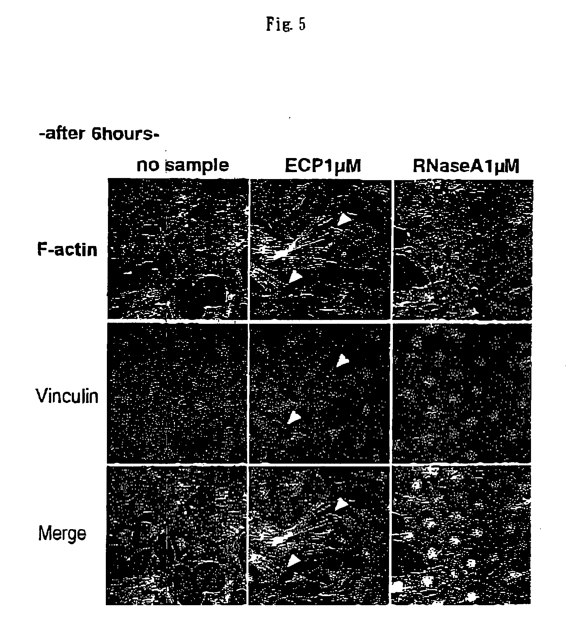

The results are as shown in Fig. 5. With regard to the cell group in the case of adding ECP, increase in the formation of many cytoskeletal molecules (stress fibers) was confirmed (indicated with an arrow).

In addition, by adding 60 pM bFGF as well as 1 µM ECP or RNaseA, observation of cytoskeleton formation was carried out in the same manner.

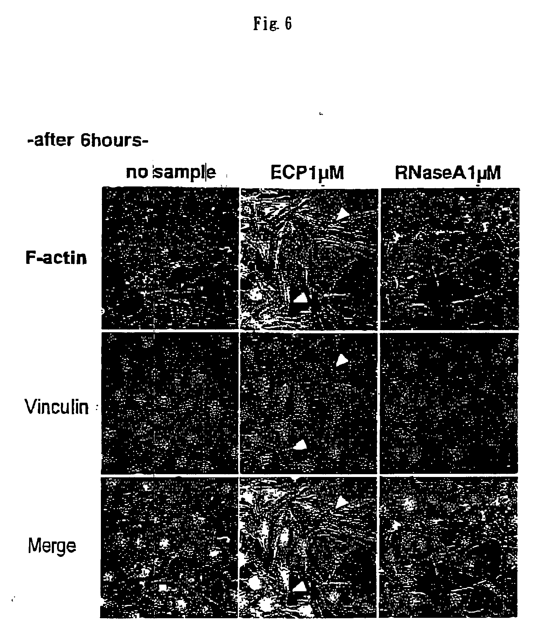

The results are as shown in Fig. 6. It was confirmed that cytoskeletal molecules were more significantly formed in the presence of ECP and bFGF.

### Example 6: Effects of ROCK inhibitor on cytoskeleton formation by ECP

BALB/3T3 cells and A31-11-1 cells which is a clone of BALB/3T3 were inoculated at a concentration of 1 x 10⁴ cells/well, and cultured in a 24-well culture plate. After 24 hours, the medium was changed to DMEM culture medium containing 0.5% FBS, and the culture was continued. After an additional 24 hours, 10 µM ROCK inhibitor (Y27632) was added as well as 1 µM ECP or RNaseA. After 6 hours, the cells were recovered and immunostained, and cytoskeleton formation was observed with a microscope (x 40 objective lens).

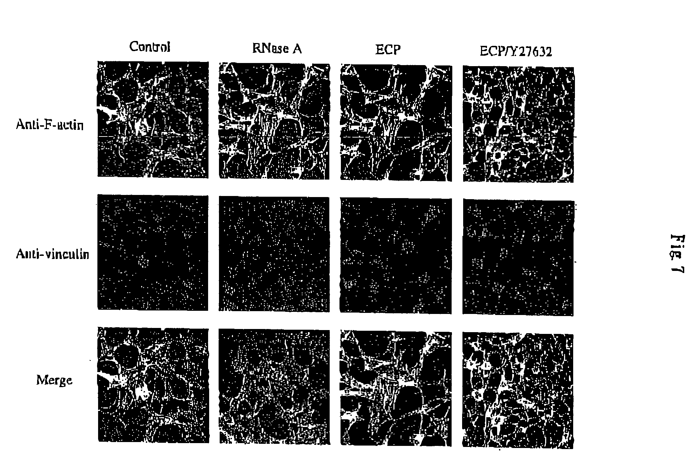

The results are as shown in Fig. 7. The promotion of stress fiber formation by ECP was inhibited by adding the ROCK inhibitor, therefore, it was confirmed that Rho-kinase was involved in cytoskeleton formation by ECP.

### Example 7: Effects of ECP on myocardial cell derived from newborn rat

The cultured myocardial cells were cultured in SFM (2 µM BrDU, 100 units/ml penicillin, 100 µg/ml streptomycin) for 48 hours, whereby the cells were dedifferentiated. Then, the culture medium was changed, and ECP was added to a final concentration of 100 ng/ml. After an additional 24 hours, the cells were washed three times with 1x PBS for 10 minutes each, then dipped in 4% paraformaldehyde at room temperature for 30 minutes, whereby the cells were fixed. Then, the cells were dipped in PBS containing 0.25% Triton-X100 at room temperature for 15 minutes, and reacted in a blocking buffer containing BSA, to which anti-ENH1 antibody had been added at a ratio of 1:40, or anti-α-actinin antibody had been added at a ratio of 1:100, at 4°C overnight. Then, a 20-minute washing with PBS containing 0.03% Triton-X100 was performed and the washing was repeated three times. To the blocking buffer, Cy3-labeled anti-mouse antibody and Cy2-labeled anti-rabbit IgG antibody were added (1:250 dilution in blocking buffer), respectively, and reaction was performed at 4°C for 4 hours. After the reaction, a 20-minute washing with PBS containing 0.03% Triton-X100 was performed again and the washing was repeated three times. Then, water content was removed and 90% glycerol was added.

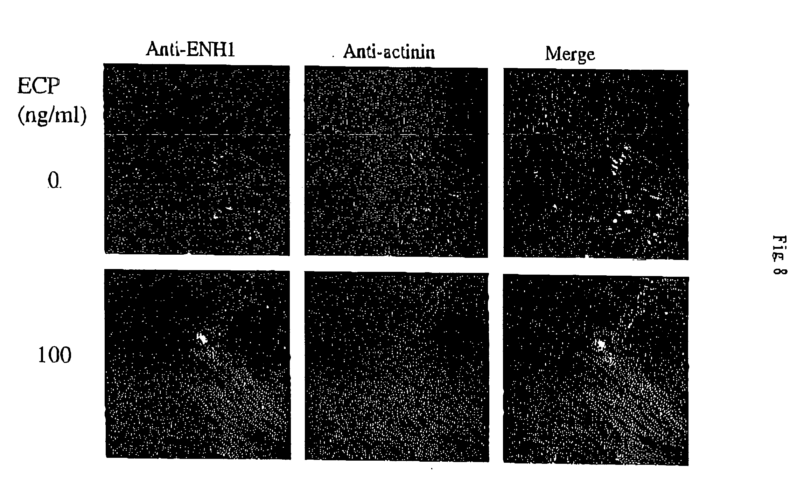

Fig. 8 shows the results of immunofluorescence staining. As shown in Fig. 8, an effect of ECP on cardiac hypertrophy in a myocardial cell was confirmed.

### Example 8: Effects of ECP on the number of heartbeats of myocardial cell derived from newborn rat

Myocardial cells isolated from a newborn rat were dedifferentiated in the same manner as in Example 7 and ECP was added to the culture solution (10 ng, 100 ng or 1 µg per ml). Twenty-four hours after the addition, the number of heartbeats was counted.

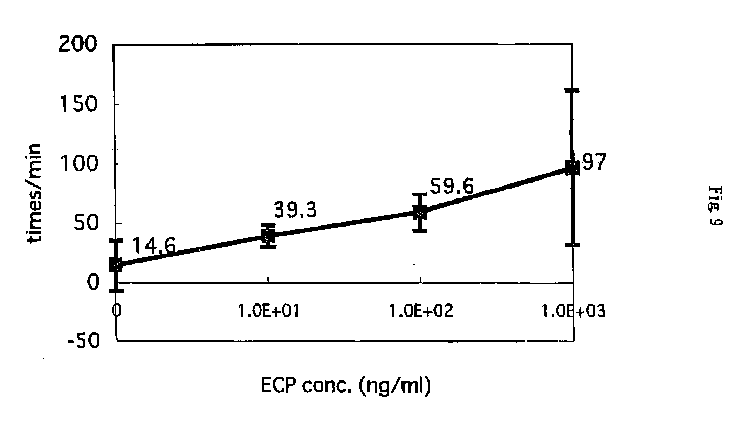

The results are as shown in Fig. 9. It was confirmed that the number of heartbeats of myocardial cell was increased in a dose-dependent manner by ECP.

Further, the effects of a ROCK inhibitor (Y27632) on the effects of ECP on increase in the number of heartbeats of myocardial cell were studied. The adding amounts of Y27632 were set at 0, 5, 10 and 15 µM, and the ECP concentration was set at 100 ng/ml.

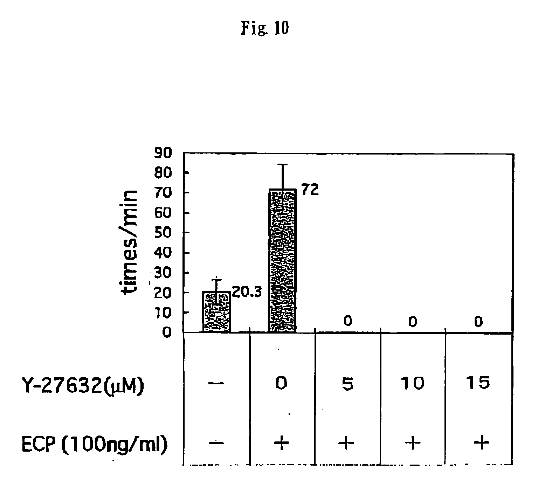

The results are as shown in Fig. 10. The increase in the number of heartbeats due to ECP was not observed by adding the ROCK inhibitor, therefore, it was confirmed that this ECP activity depended on the Rho-kinase pathway in the signal transduction cascade.

### Example 9: Effects of ECP on the survival of nerve like PC12 cell under condition of serum-free medium

PC12 cells were cultured overnight in a 96-well culture plate at 1 x 10⁴ cells/well/100 µl. As the culture medium, DMEM culture medium containing 10% FBS, penicillin, and streptomycin was used. Then, washing was performed once with 150 µl of DMEM, and the medium was changed to DMEM culture medium containing 100 µl of ECP, penicillin, and streptomycin. After cells were cultured for 72 hours, the number of living cells was counted using Cell Titer-GloTM Luminescent Cell Viability Assay (No. G7570; Promega).

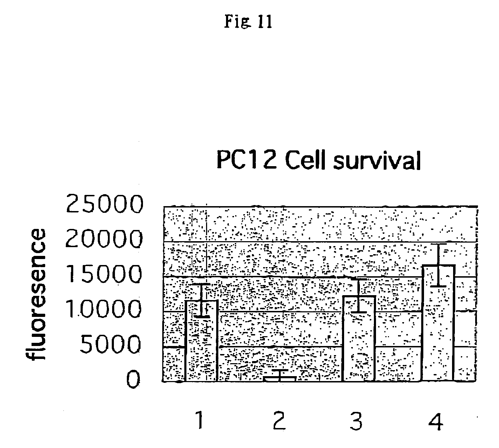

The results are as shown Fig. 11. It was confirmed that by adding ECP, the survival of nerve cells was maintained.

### Example 10: Effects of ECP on alkali phosphatase (ALP) activity in osteoblast-like MC3T3-E1 cell

MC3T3-E1 cells were inoculated into α-MEM medium in a 24-well culture plate, and ECP (at a final concentration of 1 ng, 10 ng, 100 ng or 1 µg per ml), BMP-4 (100 ng/ml) or bFGF (100 ng/ml) was added. After 72 hours, the cells were washed with 10 mM Tris-HCl (pH 7.2) solution twice, and lysed in 10 mM Tris-HCl (pH 7.2) solution containing 0.1% Triton-X100. Then, the cell lysis solution was subjected to sonication treatment for 15 seconds to homoginize the cells. Then, 100 µl of the homogenized cell solution and 0.1 M aminomethyl propanol were mixed with 100 µl of a substrate solution (4 mg/ml p-nitorophenyl phosphate and 2 mM MgCl₂) adjusted at pH 10.5, and they were reacted with each other at 37°C for 30 minutes. The reaction was terminated with 0.5 M NaOH, and the absorbance at 410 nm was measured. The obtained value was divided by the total amount of proteins in the homogenized cell solution, whereby the specific activity was calculated.

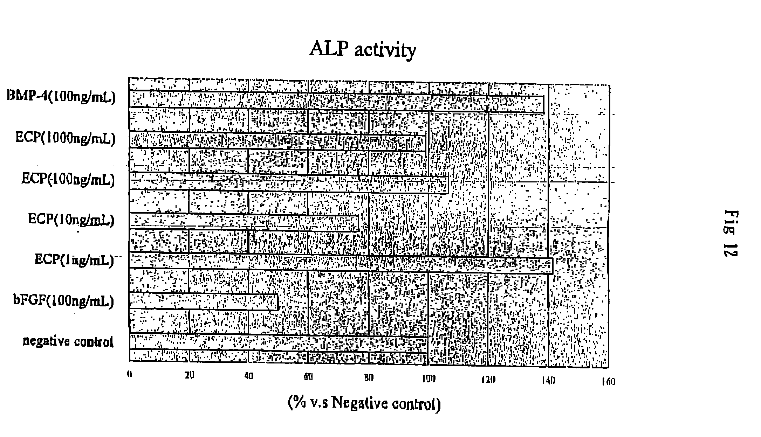

The results are as shown in Fig. 12. The value of the control was defined as 100%, and the effects of the respective added components are shown. In the case of adding ECP at 1 ng/ml, the ALP activity equal to or higher than that of BMP-4 which is a bone-forming factor was obtained.

### Industrial Applicability

As described in detail above, according to the invention of this application, a therapeutic composition based on a novel physiological activity of ECP is provided, and a new way to cure a heart disease, bone disease, neurodegenerative disease or the like caused by an aberration in the survival, proliferation and/or differentiation of a cell will be developed. In addition, a medium composition for prolonging the survival time, promoting proliferation and/or promoting differentiation of a cultured cell is provided. Further, a method for specifying a novel factor for promoting the survival, proliferation and/or differentiation of a cell is provided, whereby it becomes possible to develop a more effective therapeutic composition.

## Claims

1. A composition which is a therapeutic composition for a disease caused by a failure in the survival, proliferation and/or differentiation of a cell **characterized by** containing eosinophil cationic protein and pharmacological components.

2. The composition according to claim 1, wherein the disease caused by a failure in the survival, proliferation and/or differentiation of a cell is a heart disease, bone disease or neurodegenerative disease.

3. A composition which is a medium composition for promoting the survival, proliferation and/or differentiation of a cell containing eosinophil cationic protein and a cell biological component.

4. A screening method, which is a method of screening an active ingredient substance of a therapeutic composition for a disease caused by a failure in the survival, proliferation and/or differentiation of a cell, **characterized by** bringing a candidate substance into contact with a cell and specifying, as a target substance, a substance for promoting the survival and/or differentiation of a cell at the same level or higher than eosinophil cationic protein.

5. The screening method according to claim 4, wherein the cell is a nerve cell, bone cell, myocardial cell or fibroblast.
